Europäisches Patentamt

(19) European Patent Office

Office européen des brevets

(11) Publication number : **0 173 668 B2**

## (12) NEW EUROPEAN PATENT SPECIFICATION

(45) Date of publication of the new patent specification : **15.07.92 Bulletin 92/29**

(21) Application number : **85870119.6**

(22) Date of filing : **28.08.85**

(51) Int. Cl.$^5$ : **C12N 15/63, C12P 21/02, C12N 1/16, // (C12R1/85, 1:865, 1:72, 1:725, 1:74, 1:19)**

(54) Recombinant saccharomyces.

(30) Priority : **31.08.84 US 645973**

(43) Date of publication of application :
**05.03.86 Bulletin 86/10**

(45) Publication of the grant of the patent :
**31.05.89 Bulletin 89/22**

(45) Mention of the opposition decision :
**15.07.92 Bulletin 92/29**

(84) Designated Contracting States :
**AT BE CH DE FR GB IT LI LU NL SE**

(56) References cited :
**EP-A- 0 103 201**
**EP-A- 0 129 268**
**NATURE,vol.289, no. 5793, 1/8 January 1981, New York, London, S. HENIKOFF et al. "Isolation of a gene from Drosophila bycomplementation in yeast", pp. 33-37**
**GENE, vol. 10, no. 1, June 1980, Amsterdam, R.D. DICKSON, "Expression of a foreign eukaryotic gene in Saccharomycescerevisiae: beta-galactosidase from Kluyveromyces lactis", pp. 347-356**

(56) References cited :
**THE JOURNAL OF BIOLOGICAL CHEMISTRY, vol. 258, no. 1, January 10, 1983, Baltimore, USA, P.R. RUSSELL et al. "The PrimaryStrucutre of the Alsohol Dehydrogenase Gene from the Fission Yeast Schizosaccharomyces pombe", pp. 143-149**
**Gene. vol.29, 1983, pp. 157-162**
**Journal of Bacteriology, 1983, vol. 154, pp. 1033-1039**
**US Environmental Protection Agency R&D.(REP). EPA 600/9-84/015 pp. 274-287**
**Mol. Gen. Genet. 1984, vol. 198pp. 179-182**

(73) Proprietor : **SMITHKLINE BEECHAM CORPORATION**
**P.O. Box 7929 1 Franklin Plaza**
**Philadelphia Pennsylvania 19101 (US)**

(72) Inventor : **Gorman, Jessica Angell**
**152 Ridgefield Road**
**Newtown Square, PA 19073 (US)**
Inventor : **Koltin, Yigal**
**P.O. Box 7929**
**Philadelphia, PA 19101 (US)**

(74) Representative : **Valentine, Jill Barbara et al**
**SmithKline Beecham Corporate Patents Great Burgh Yew Tree Bottom Road**
**Epsom Surrey KT18 5XQ (GB)**

EP 0 173 668 B2

## Description

### BACKGROUND OF THE INVENTION

This invention relates to cloning and expression of DNA sequences from Candida Albicans in Saccharomyces.

Candidiasis is an infection caused by any of several species of the yeast genus Candida. These organisms are members of the normal flora of the skin, mucous membranes, and gastrointestinal tract. The risk of endogenous infection is, thus, clearly ever present. Most Candida infections are caused by Candida albicans which is a common human fungal pathogen that causes both superficial and systemic mycosis. Candida albicans infections are most often associated with immune compromised patients. In such patients, the infection can be life threatening. Therefore, there is a real need for anti-candidial agents. The isolation and expression of Candida genes may enable the discovery of effective anti-candidial agents.

The understanding of the biochemistry and genetics of Candida has been hampered by the lack of an amenable genetic system. It is difficult to prepare and isolate mutants of Candida, in which no sexual stage has been described. In the absence of suitable mutants, it is difficult to analyze the biochemical and developmental pathways of Candida. Thus, the development of effective anti-candidial agents has been slow.

Saccharomyces cerevisiae is considered largely non-pathogenic and is a favorite organism for cloning and expression of recombinant DNA. Struhl, Nature, 305: 391-397 (1983), reviews cloning in Saccharomyces. Henikoff et al., Nature 289:33-37 (1981), report the isolation of an adenine-8 gene from Drosophila by complementation in yeast. Dickson, Gene, 10;347-356 (1980), reports the expression of a β-galactosidase gene derived from Kluyveromyces lactis in Saccharomyces cerevisiae. Russell and Hall, J. Biol. Chem., 258:143-149 (1983), report the cloning of an alcohol dehydrogenase gene from Schizosaccharomyces pombe in Saccharomyces cerevisiae. Jellinghaus et al., J. Mol. Biol., 159:623-636 (1982), report the transcription in yeast of a Dictyostelium gene cloned in an Escherichia coli-yeast vector.

### SUMMARY OF THE INVENTION

One aspect of this invention is a process for preparing a transformed host microorganism of the genus Sacchromyces which transforming said host microorganism with comprises a functional DNA sequence derived from Candida albicans, wherein the functional DNA sequence comprises a complete gene expression unit, a structural gene, a promoter, or a regulators region.

Another aspect of this invention is a process for preparing a recombinant plasmid comprising a replicator region, and a functional DNA sequence derived from Candida albicans, wherein the functional DNA sequence comprises a complete gene expression unit, a structural gene, a promoter, or a regulators region.

This invention also relates to a method of expressing a functional DNA sequence derived from Candida albicans which comprises-transforming a host microorganism of the genus Saccharomyces with the functional DNA sequence, and culturing the transformed host under suitable conditions such that the functional DNA sequence is expressed, wherein the functional DNA sequence comprises a complete gene expression unit, a structural gene, a promoter, or a regulators region.

Yet another aspect of this invention is a method of characterizing a DNA sequence derived from Candida albicans by its function which comprises (a) transforming a host microorganism of the genus Saccharomyces with the DNA sequence, wherein said host microorganism lacks a known function, so that the Candida albicans DNA can complement the most microorganism; and (b) assaying for presence of the function in the transformed host microorganism.

This invention also relates to a method of producing a polypeptide in Saccharomyces which comprises culturing a transformed host microorganism of the genus Saccharomyces, wherein said host microorganism comprises a promoter derived from Candida albicans fused, directly or indirectly, in phase and in proper orientation to a structural gene which codes for the polypeptide.

### DETAILED DESCRIPTION OF THE INVENTION

The transformed host microorganism the preparation of which is the subject invention is any member of the genus Saccharomyces into, which a functional DNA sequence derived from Candida albicans has been introduced. Saccharomyces cerevisiae is preferred because it is the most well studied member of the genus and has been the species most frequently employed for recombinant DNA purposes.

The recombinant plasmid the preparation of which is the subject invention comprises a functional DNA sequence derived from any species of the genus Candida albicans.

By the term "functional DNA sequence" is meant any discrete region of DNA derived directly or indirectly from Candida albicans which functions in Candida albicans as a complete gene expression permit, a structural gene, a promoter, or a regulatory region. By "complete gene expression unit" is meant a structural gene and the promoter and regulatory regions required for its transcription and translation. By "promoter" is meant any region upstream of a structural gene which permits binding of RNA polymerase and transcription to occur. By "regulatory regions" is meant any region which regulates transcription of the gene. By "structural gene" is meant a coding sequence for a polypeptide which serves to be the template for the synthesis of mRNA.

The recombinant plasmid the preparation of which is the subject invention also comprises a replicator region, preferably a Saccharomyces or Candida albicans replicator region. By "replicator regions" is meant any DNA fragment, preferably derived directly or indirectly from yeast, such as Saccharomyces or Candida albicans which can be use to maintain, extrachromosomally, a functional DNA sequence derived from Candida albicans in the host Saccharomyces microorganism. Particularly preferred plasmids are those comprising a Saccharomyces-Escherichia coli shuttle vector, such as the one described by Broach et al., Gene, 8:121-133 (1979). Illustrative of vectors which can be used in this invention by cloning therein a functional DNA sequence derived from Candida albicans are the following plasmids which are publicly available, e.g., from the American Type Culture Collection, Rockville, Maryland, U.S.A. (ATCC® accession, number in parentheses): pBTI-1 (37087), pBTI-7 (37088), pBTI-9 (37089), pBTI-10 (37090), pLC544 (37013), pRB3 (37059), pRB4 (37058), pRB5 (37051), pRB8 (37109), pRB9 (37057), pRB10 (37056), pRB18 (37092), pRB20 (37054), pRB21 (37055), pRB22 (37053), pRB12 (37061), pRB14 (37063), pRB15 (37062), pRB16 (37060), pRB19 (37064), YEp2 (37076), YRp17 (37078), YEp13 (37115).

The Candida albicans DNA sequence can be prepared by known techniques such as the one described by Rigsby et al., Molec. Cell. Biol., 2:853-862 (1982). The isolated Candida albicans DNA can then be further fragmented by treatment with one or more restriction enzymes.

A recombinant plasmid containing a functional Candida albicans DNA sequence and containing an Escherichia coli replicator region can be used to transform Escherichia coli to amplify the plasmid. Such transformation and amplification can be accomplished by known techniques. The recombinant plasmid is then retrieved from E. coli by known techniques.

The retrieved recombinant plasmid can then be used to transform a host microorganism belonging to the genus Saccharomyces. Such transformation can be accomplished by the random insertion of recombinant plasmid DNA fragments into covalently closed circular yeast plasmids and (a) transforming spheroplasts therewith, such as by the method described by Hinnen et al., Proc. Nat'l. Acad. Sci. USA, 75:1929-1933 (1978); or (b) by using the lithium acetate or lithium chloride method of yeast transformation, such as described by Ito et al., J. Bacteriol., 153:163(1983).

DNA from Candida albicans can also be transformed into a Saccharomyces microorganism by fusion of Candida albicans and Saccharomyces protoplasts. The introduced Candida albicans DNA can be integrated into the host Saccharomyces microorganism's chromosomal DNA, such as by recombination, or can remain extrachromosomal. Thus, yet another exemplary method is transformation of host Saccharomyces organism directly with Candida albicans DNA which, optionally, has flanking sequences which are homologous to a contiguous sequence in the host's chromosomal DNA thereby permitting recombination with the host's chromosomal DNA.

Promoter regions derived from Candida albicans by the process of the subject invention can be used to express coding sequences from Candida, Saccharomyces or other organisms, viruses or cells in the transformed Saccharomyces host. Such promoters can be fused to a structural gene, such as sequences coding for insulin, interferon, growth hormone, etc., directly by fusing all or a portion of the promoter's natural nucleotide to the coding sequence for the desired polypeptide, thereby producing a hybrid gene. When Saccharomyces host microorganisms, which have been transformed by a plasmid containing such hybrid gene, are cultured in suitable culturing media, they produce the polypeptide. In addition to those Candida albicans promoters disclosed herein, additional promoters can be discovered by cloning random fragments of Candida albicans DNA in Saccharomyces plasmids designed for promoter selection. Such plasmids can be designed by known techniques such as the one described by Casadaban et al., Methods in Enzymology, 100:293-308(1983).

Strutural genes derived from Candida albicans can be expressed in Saccharomyces from their natural Candida albicans promoters, from heterologous promoters derived from Candida or Saccharomyces, or from other functional heterologous promoters which are derived from other organisms or produced from synthetic oligonucleotides and which function in Saccharomyces.

Many known Saccharomyces mutants are defective in defined functions, such as a specific step in leucine or histidine biosynthesis. This function could be supplied if such mutants were transformed with DNA sequences derived from Candida albicans which contain genes coding for such functions. Where the function to be characterized is not already defective in an available Saccharomyces strain, such mutations can be induced by known techniques, such as by ultraviolet radiation, chemical mutagenesis, or genetic manipulation. In some cases, the function displayed by the transformed host microorganism will be one rich is naturally not present in Saccharomyces. For example, a host Saccharomyces cerevisiae microorganism can be transformed by a functional DNA sequence according to the method of this invention to display functions it does not naturally possess such as sorbitol utilization, maltose utilization by isomaltase catalyzed cleavage of $\alpha$ -1-6-glucosidic bonds (such as $\alpha$ -methyl-D glucopyranoside hydrolysis), soluble starch utilization by glucoamylase catalyzed cleavage of $\alpha$ -1-4-glucosidic bonds or resistance to an anti-fungal agent.

The development of a genomic library covering the entire genome of Candida albicans enable the characterization of the functions of the DNA derived from Candida albicans. This will permit the genetics of this asexual pathogen to be more closely analyzed than has been possible in the past and to facilitate the development of anti-candidal agents. The preferred method of characterizing the functions of DNA derived from Candida albicans is by expressing DNA derived from Candida albicans in Saccharomyces cerevisiae so that the Candida albicans genes can be studied in the background of a well characterized eukaryotic organism. Thus, the genetics of Candida albicans can be studied by isolation of genes via complementation of functions displayed by transformed Saccharomyces cerevisiae. The abundance of available Saccharomyces cerevisiae mutant strains, together with the posiblility of selection of additional mutants, makes it possible to effect such complementation. Therefore, it is possible to select for Candida albicans genes associated with any one of a number of functions, such as those associated with defined biochemical reactions, on those involved in cell division on cell structure. The availability of the genomic library and a system for the identification of the selected genes offers a new approach to resolve some of the basic differences that make one orgamism economically useful and another orgamism a serious pathogen.

Using the method of this invention, plasmids carrying one on more functional C. albicans genes which complement a Saccharomyces mutation on confer a new characteristic to the Saccharomyces strain transformed therewith can be isolated and characterized. Such functional C. albicans genes can include a gene complementing the S. cerevisiae trpl mutation, a gene complementing the S. cerevisiae his3 mutation, a gene conferring high level resistance to the anti-fungal agent benomyl, a gene allowing for growth on sorbitol, a gene allowing for growth on isomaltose, a gene allowing for growth on soluble starch (i.e., a glucoamylase gene), and a gene encoding the enzyme galactokinase which complements a S. cerevisiae gall mutation. Such genes are useful as markers for isolating Saccharomyces hosts successfully transformed with plasmids containing such genes. Each of the promoters of the C. albicans sorbitol, isomaltose and soluble stanch utilization genes is also useful as a regulatable promoter for a Saccharomyces microorganism transformed with a plasmid containing such a promoter. Plasmids containing C. albicans functional DNA sequences are useful for production in Saccharomyces of gene products, such as pharmaceuticals, vaccinal antigens and hormones.

In the following examples, specific embodiments of the invention are more fully disclosed. These examples are intended to be illustrative of the subject invention and should not be construed as limiting its scope.

**Example 1**

A. Isolation of DNA from C. albicans

The DNA from C. albicans strain 792-1 (courtesy of J. Kwon-Chung, Natl. Inst. Health, U.S.A.) was isolated by the procedure described by Rigsby et al., Molec. Cell Biol., 2: 853 - 862 (1982). The DNA purified by this procedure was on the average 100 kilobase pains (kbp) in length.

B. Construction of the genomic library of C. albicans

A genomic library was constructed in the Saccharomyces-Escherichia coli shuttle vector, YEp13, described by Broach et al., Gene, 8: 121 - 133 (1979). Calculation of the size of the genome of C. albicans based on the data provided by Rigsby et al., cited above, suggests that the genome size of C. albicans is about $2 \times 10^4$ kbp. Therefone, some 8000 independent transformants carrying an insert of 10 kbp are required to provide a genomic library with a probability of 99 % that each of the sequences of C. albicans will be represented at least once in this library. This was calculated by the method disclosed by Maniatis et al., "Molecular cloning - A Laboratory Manual", Cold Spring Harbor Laboratory (1982).

The DNA was partially digested with the restriction endonuclease Sau3A and fractionated by centrifugation in a 5 - 20 % sucrose gradient. The fractions containing DNA fragments in the range of 10 kbp were pooled and used for ligation. Plasmid YEp13 DNA was cleaved at the unique BamHI site, and then treated with alkaline phosphatase to prevent self-ligation. Equal concentrations (200 ng. in a 10 µL reaction mixture) of C. albicans and YEp13 DNA were ligated.

E. coli strains MC1061 (Casadaban et al., J. Mol. Biol., 138: 179 - 207 (1980)) and HB101 (Bolivar et al., Gene, 2: 95 - 113 (1979)) were transformed with the ligated mixture according to Lederberg and Cohen, J. Bacteriol., 119: 1072 - 1074 (1974), with the modification that a 45 min. expression time at 37°C in LB (Luria Broth) was allowed. An estimate of the number of independent transformants was obtained by plating a small sample on LB plates containing 50 mcg/ml of ampicillin. Simultaneously, the remaining sample was inoculated into LB broth containing 50 mcg/ml of ampicillin and incubated overnight to use for DNA isolation. The plate results indicated that some 16 000 independent clones were obtained. To estimate the percentage of transformants which contained inserts, 100 transformants were tested for thein resistance to ampicillin and to tetracycline. Since the BamHI site is in the tetracycline resistance gene (tet$^R$), transformants that contain plasmids with the inserted DNA should be sensitive to tetracycline and resistant to ampicillin, while transformants containing plasmids without inserts should be resistant to both antibiotics. The results obtained indicated that all the transformants were sensitive to tetracycline and resistant to ampicillin suggesting that at least 99 % of the transformants were carrying plasmids containing inserted DNA of C. albicans.

DNA was prepared from the overnight LB-ampicillin culture of E. coli, either small scale or large scale by the method of Maniatis et al., cited above, using an aliquot of this original culture as inoculum.

C. Expression of C. albicans genes in S. cerevisiae

1. General methodology

To isolate cloned DNA sequences of C. albicans by expression in S. cerevisiae, genetically defined haploid strains of S.cerevisiae were employed as hosts for DNA mediated transformation. Transformation was performed a) by the spheroplast procedure, as described by Sherman, cited above, or by the lithium chloride or lithium acetate method described by Ito et al., J. Bacteriol., 153: 163 (1983). The yeast were plated on minimal medium (Yeast nitrogen base-Difco YNB) containing all necessary supplements except one.

Transformation of a defined S. cerevisiae mutant with a plasmid containing the wild type allele of the specific mutation allows the transformed host to grow under conditions restrictive to the non-transformed mutant. In the examples following the LEU2 gene was used as the first selectable marker. The transformed yeast were then subcultured onto additional types of media to select transformants that expressed other functions originally lacking in the S. cerevisiae host strain.

The plasmids were recovered from S. cerevisiae by making a DNA preparation of the desired transformant, using the procedure of Sherman et al., cited above, and using this DNA to transform E. coli. Since the shuttle vector carried the ampicillin resistance gene, transformed E. coli were recoveced on LB-ampicillin plates. Plasmid DNA was then isolated from the E. coli by known techniques and used for further analysis. Such further study included:

a) transformation of yeast to confirm the presence of a gene conferring the same phenotype as found in the original transformed yeast cell;
b) hybridization analysis to conhirm the presence

of DNA of C. albicans in the hybrid plasmid; and
c) further analysis of the C. albicans DNA fragment both structurally and functionally.

2. Isolation of a Candida albicans gene that complements the trpI mutation of S. cerevisiae

Spheroplasts of S. cerevisiae strain DBY746 ($\alpha$, his3-1 leu2-3 leu2-112, ura3-52, trpI-289) were transformed with DNA isolated from the C. albicans library. S. cerevisiae strain DBY746 was obtained from yeast Genetic Stock center, Department of Biophysics and Medical physics, University of california, Berkeley, California, U.S.A. The cells were plated on minimal medium (Difco yeast nitrogen base) containing 011 required supplements except leucine. The transformed yeast cells expressed the plasmid encoded LEU2 gene. The Leu $^+$ cells were harvested from these plates and plated onto minimal medium lacking both leucine and tryptophane. Of the 500 transformants examined, two grew on these plates.

To test whether the growth of these two transformants on medium lacking leucine and tryptophane was due to the presence of a C. albicans DNA insert, rather than due to the reversion of the trpI mutation, the transformants were grown in non-selective yeast extract peptone dextrose (YEPD) medium for 10 generations, and then plated on minimal medium containing all supplements. The colonies were then replica plated onto minimal medium lacking only tryptophane or only leucine.

In all cases, colonies arising from cells that had lost the plasmid, i.e., were Leu$^-$, also required tryptophane indicating co'segregation of the LEU2 and TRP1 alleles.

Plasmids were recovered from the two transformed isolates by transformation of E. coli HB101 with small yeast DNA preparations prepared by the method of Sherman et al., "Methods in Yeast Genetics Laboratory Manual", Cold Spring Harbor Laboratory (1982) selecting for ampicillin resistant transformants. The transformed E. coli cells were tetracycline sensitive, indicating that the plasmid contained an insert in the tet$^R$ gene. The two plasmids were designated pAR84-1 and pAR84-2.

Plasmid DNA of each was prepared by the method of Maniatis et al., cited above, and used to transform S. cerevisiae strain DBY746 (available from Yeast Genetic Stock center). The selection of the transformed cells was performed on medium devoid of leucine but containing the supplements histidine, tryptophane and uracil. The Leu$^+$ transformed cells were tested for prototrophy for tryptophane. Between 88 to 100 % of the transformants expressed the unselected marker TRP$^+$ confirming that both pAR84-1 and pAR84-2 contained a gene complementing the S cerevisiae trpI mgtation and indicating that a functional DNA sequence derived from Candida can be expressed in Saccharomyces.

D. Further characterization of the plasmids

To test for specific hybridization of the DNA insert in the recombinant plasmids to genomic DNA of C. albicans, DNA from non-transformed and transformed S. cerevisiae strain DBY746 was isolated according to the procedune described by Sherman et al., cited above, for small scale preparations. The DNA of E. coli, with or without plasmid, was also prepared by the method of Maniatis et al., cited above.

The DNA samples were digested with the restriction enzymes HindIII and BglII. Digested DNA of C. albicans strain 792-1, S. cerevisiae strain DBY746, plasmid YEp13, and plasmid pAR84-2 was electrophoresed on agarose gels, and transferred to a nitrocellulose filter by the procedure of Southern, J. Mol. Biol., 98: 503-517 (1975). The filter was then hybridized sequentially, first with $^{32}$p labelled YEp13 DNA to test whether the vector used hybridized to the genomic DNA of C. albicans, and, second, with $^{32}$p labelled pAR84-2 DNA to determine whether the new plasmid hybridized to the genomic DNA of C. albicans.

The nitrocellulose filters were prehybridized for 3 hrs. at 65° C with a solution containing 6 x SSC and 3 x Denhardt (1 x Denhardt is 0-02 % Ficol 400, 0.02 % polyvinylpyrrolidone 360 and 0.02 % bovine serum albumin) according to the method of Maniatis et al. cited above. Hybridization solution was the same except the $^{32}$p labelled DNA probe prepared by nick translation (according to the method of Maniatis et al., cited above) was added. Hybridization was performed for 18 hours at 65° C. After hybridization the filters were washed with 2 x SSC at 65° C. The filters were then exposed to X-ray film.

YEp13 DNA hybridized only to the genomic DNA of S. cerevisiae, whereas pAR84-2 DNA also hybridized to the genomic DNA of C. albicans. The hybridization of pAR84-2 to the S. cerevisiae genomic DNA is due to the S. cerevisiae LEU2 gene that is a part of the plasmid. The hybridization to C. albicans is due to homology of Candida insert with genomic sequences of Candida.

The size of the inserts in each plasmid was estimated by electrophoresis of restriction digests on agarose gels. pAR84-1 contains a 15 kbp fragment of C. albicans DNA, while pAR84-2 contains a 10.4 kbp fragment.

E. Other Specific Examples

Using the same methodology as that used for the isolation of a C. albicans DNA fragment that complements the S. cerevisiae trpl mutation, plasmids carrying other functional C. albicans genes have been isolated and characterized.

These include:

1) A gene complementing the his3 mutation.

2) A gene confenring high level resistance to the anti-fungal agent benomyl (methyl 1- (butylcarbamoyl)-2-benzimidazole- carbamate). Strains of C. albicans are more resistant to benomyl than S. cerevisiae. This gene thus confers a new characteristic to the transformed S. cerevisiae strain.

3) A gene allowing for growth on the carbon source sorbitol. Strains of C. albicans can grow on sorbitol but S. cerevisiae and other species of Saccharomyces cannot utilize this sugan. This gene thus confers a new characteristic to the transformed S. cerevisiae strain.

4) A gene allowing for growth on the carbon source αmethyl-D-glucopynanoside (isomaltose). Strains of C. albicans can grow on this substrate but most strains of S. cerevisiae cannot naturally utilize this sugar. This enzyme has been found in other species of Saccharomyces. Thus, the C. albicans gene which codes for isomaltase (which cleaves α-1-6 glucosidic bonds) confers a new characteristic to the transformed S. cerevisiae host.

5) A gene coding for an enzyme allowing for growth on soluble starch. Strains of C. albicans can utilize such soluble starch as a substrate but S. cerevisiae cannot naturally utilize this carbon source. This enzyme, glucoamylase, has been found in other species of Saccharomyces. Thus, the C. albicans gene which codes for glucoamylase (which cleaves α-1-4 glucosidic bonds) confers a new characteristic to the transformed S. cerevisiae host. Furthermore, the C. albicans glucoamylase gene is of great potential utility, as the enzyme, when produced by C. albicans on by a S. cerevisiae host transformed therewith, is excreted into the medium. Thus, the glucoamylase gene may provide DNA sequences for a signal peptide which functions in protein secretion. The use of such a signal peptide for secretion of a cytoplasmic protein is well known, for example, see Silhavy et al., U.S. Patent 4 335 336, issued June 22, 1982. This glucoamylase gene signal peptide can be used to develop a system for secretion of natural or heterologous proteins expressed by Sacchanomyces or other yeast transformed therewith.

6) A gene encoding the enzyme galactokinase, which complements a S. cerevisiae gall mutation.

## Example 2

Complementation of the S. cerevisiae his3 gene
with C. albicans DNA

a. Isolation of a C. albicans gene that complements
the his3 mutation of S. cerevisiae

The his3 locus of S. cerevisiae encodes
imidazoleglycenol phosphate dehydrogenase. The
isolation of a C. albicans gene that complements the
his3 mutation of S. cerevisiae was carried out according to the procedure of Example 1. The Leu $^+$ yeast
transformants were plated on medium lacking leucine
and histidine Colonies which arose were selected.

b. Recovery of a plasmid containing a C. albicans
gene that complements the his3 mutation of S.
cerevisiae

Plasmids were recovered from the selected colonies, described above, by transformation of E. coli
HB101 according to the method of Example 1. Plasmid DNA was isolated from the E. coli transformants
according to the method of Example 1, and was designated pAR84-3.

c. Transformation of S. cerevisiae strain DBY746
with a plasmid containing a C. albicans gene that
complements the his3 mutation of S. cerevisiae

Plasmid pAR84-3, prepared as described above,
was transformed into S. cerevisiae strain DBY746
according to the procedure of Example 1.

d. Selection of transformants and confirmation of
the presence of C. albicans DNA in the transformant

Host S. cerevisiae microorganisms, transformed
with plasmid pAR84-3 according to the method described above, were selected according to the method
described above, and tested for expression of the his3
gene.

## Example 3

Expression of the C. albicans benomyl resistance
gene in S. cerevisiae

The isolation of the C. albicans benomyl resitance
gene was carried out according to the procedure of
Example 1, except that S. cerevisiae strain JWG2-2C
(α, leu2-3, leu2-112) was employed as the host. S.
cerevisiae strain JWG2-2C was derived from across
of S. cerevisiae strain DC5 (leu2-3, leu2-112, his3,
ga12, canl-11), with S. cerevisiae strain YM146 (a,
gall Δ152, trpl- 289, ura3-52, rho⁻). S. cerevisiae
strain DC5 was obtained from James R. Broach, S.U.N.Y., Stonybrook, New York U.S.A. S. cerevisiae
strain YM146 was obtained from Mark Johnson,
Washington State University, pullman, Washington,
U.S.A. The Leu⁺ transformed cells were plated on
miminal medium (Difco yeast nitrogen base) containing all required supplements except leucine, and also
containing benomyl (50 μg/ml). Several benomyl
resistant colonies were obtained.

b. Recovery of plasmids containing the C. albicans
benomyl resistance gene

Plasmids containing a C. albicans gene that
codes for an enzyme conferring benomyl resistance
were recovered according to the procedure of
Example 1. Plasmid DNA was isolated from individual
E. coli transformants according to the small scale
method of Sherman et al., "Methods in Yeast Genetics
Laboratory Manual", cold Spring Harbor Laboratory
(1982). The size of the C. albicans insert in the recovered plasmids was estimated by agarose gel
electrophoresis.

c. Transformation of S. cerevisiae strain JWG2-11D
with a plasmid containing the C. albicans benomyl
resistance gene

The plasmid DNA was isolated from the E. coli
transformants, and used to transform S. cerevisiae
strain JWG2-11 D (α, his3, trpl-289, leu2-3, leu2-112,
gall Δ152), according to the procedure of Example 1.
S. cerevisiae strain JWG2-11D was derived by the
same method as S. cerevisiae strain JWG2-2C.

d. Selection of transformants and confirmation of
the presence of C. albicans DNA in the transformant

Host S. cerevisiae microorganisms, transformed
with a plasmid containing the C. albicans benomyl
resitance gene according to the method described
above, were selected as described above, and the
presence of plasmid in the transformed hosts was
confirmed according to the method of Example 1 by
plating on medium containing benomyl. Three independent isolates, designated pCAben17 pCAben3
and pCAben15, were further analyzed by restriction
mapping, and all were found to contain an identical
5.8 kilobase pain (kbp) DNA fragment from C. albicans.

## Example 4

Expression of the C. albicans sorbitol utilization
gene in S. cerevisiae

a. Isolation of the C. albicans sorbitol utilization
gene
The isolation of the C. albicans sorbitol utilization gene was carried out according procedure of Example 1, except that S. cerevisiae strain JWG2-2C was employed as the host. Leu⁺ transformants were plated on minimal medium (Difco yeast nitrogen base) containing all required supplements except leucine and glucose, and also containing 2 % sorbitol.

b. Recovery of plasmids containing the C. albicans sorbitol utilization gene

Plasmids containing a C. albicans gene that codes for an enzyme conferring sorbitol utilization ability were recovered according to the procedure of Example 1. Plasmid DNA was isolated from individual E. coli transformants according to the small scale method of Sherman et al., cited above, and the size of the C. albicans insert of the recovered plasmids was estimated by agarose gel electrophoresis.

c. Transformation of S. cerevisiae strain JWG2-11D with a plasmid containing the C. albicans sorbitol utilization gene

Plasmid DNA was isolated from the E. coli transformants described above, and used to transform S. cerevisiae strain JWG2-11D according to the method of Example 1.

d. Selection of transformants and confirmation of the presence of C. albicans DNA in the transformant

Host cells of C. cerevisiae, transformed with a plasmid containing the C. albicans sorbitol utilization gene according to the method described above, were selected as described above, and the presence of the plasmid in the transformed hosts was confirmed according to the method of Example 1. DNA of two independent isolates, designated pCAsor2 and pCAsor9, were further analyzed by restriction mapping- pCAsor2 was found to contain a 4.5 kbp DNA insert from C. albicans, while pCAsor9 was found to contain a 4.8 kbp DNA insert from C. albicans which showed little homology to the pCAsor2 C. albicans DNA insert.

**Example 5**

Expression of the C. albicans glucoamylase gene in S. cerevisiae

a. Isolation of the C. albicans glucoamylase gene
The isolation of the C. albicans glucoamylase gene was carried out according to the procedure of Example 1, except that S. cerevisiae strain JWG2-2C was employed as the host. Leu⁺ transformants were plated on minimal media (Difco yeast nitrogen base) containing all required supplements except leucine and glucose, and also. containing 2 % soluble starch. The E. coli transformants were probably secreting the glucoamylase since satellite colonies began to grow adjacent to the original large colonies.

b. Recovery of a plasmid containing the C. albicans glucoamylase gene

Plasmids containing the C. albicans gene that codes for glucoamylase were recovered in E. coli according to the procedure of Example 1. Plasmid DNA was isolated from individual E. coli transformants according to the method of Example 1.

c. Transformation of S. cerevisiae strain JWG2-11D with a plasmid containing the C. albicans glucoamylase gene

Plasmid DNA, isolated from the E. coli transformants as described above, was used to transform S. cerevisiae strain JWG2-11D according to the method of Example 1.

d. Selection of transformants and confirmation of the presence of C. albicans DNA in the transformant

Host S. cerevisiae microorganisms, transformed with a plasmid according to the method described above, were selected as described above, and the presence of the plasmid in the transformed host was confirmed according to the method of Example 1. Three independent isolates, designated as pSTA27-1 pSTA1-3 and pSTA15-1, were further analyzed by restriction mapping which revealed that pSTA27-1 and pSTA1-3 contain an identical C. albicans DNA insert while pSTA15-1 contains a possibly different C. albicans DNA insert.

Plasmid pSTA27-1 was introduced by transformation (Ito et al., J. Bacteriol., 153: 163 (1983) into three different S. cerevisiae strains, JCC4-11 (α, leu2⁻, his1⁻), JCC4-28 (α, leu2, his1) and JCC4-29 (α, leu2, his1). The same three strains were also transformed with the parent vector plasmid YEp13 lacking any C. albicans sequences. The growth of all six transformed strains was tested on complete (YEP) medium containing 0.5 % malto-oligossacharides (Mos) (Pfanstiel - Laboratories Inc.) as the sole carbon source. This compound, Mos, consists of a mixture of glucose polymers linked by alpha-1,4 glucosidic bonds. It is devoid of glucose, maltose and maltotriose, and thus consists of higher order oligomers which can not be used as carbon sources by Saccharomyces cerevisiae strains. This is due to the fact that: 1) these polysaccharides do not enter S. cerevisiae cells and 2) S. cerevisiae strains do not secrete an enzymatic activity (amylase on glucoamylase) capable of degrading Mos extracellularly. While all three strains transformed with plasmid YEp13 failed to grow on Mos containing medium, the same three strains carrying plasmid pSTA27-1, as well as C. albicans strain 792-1 (courtesy of J. Kwon-Chung, Natl. Inst. Health, U.S.A.) grow well on this medium. This result indicates that the C. albicans DNA fragment cloned in pSTA27-1 carries a gene encoding an enzyme capable of degrading Mos into a usable carbon source, that this gene is expressed in S. cerevisiae, and that its product of secreted into the culture medium. In order to confirm the expression and secretion of the C. albicans glucoamylase by a S. cerevisiae host transformed therewith, the culture media of all six transformed S. cerevisiae strains, as well as that of C. albicans strain 792-1, were tested for the presence of glucoamylase activity according to the method described by Searle et al., FEMS Microbiology Letters,, 111, 211 - 212 (1981). It was found that while the culture media of S. cerevisiae strains transformed with YEp13 were devoid of any detectable glucoamylase activity, the culture media of C. albicans strain 792-1 and that of S. cerevisiae containing pSTA27-1 contained appreciable amounts of glucoamylase. In fact, it was found that S. cerevisiae strains carrying plasmid pSTA27-1 secrete 15 to 30 fold more glucoamylase activity into their culture medium than does C. albicans strain 792-1.

The glucoamylase activity secreted by S. cerevisiae strains transformed with pSTA27-1 was further characterized and compared to that secreted by C. albicans strain 792-1 in the following manner:

a) The pH optimum of the enzyme secreted by S. cerevisiae strains was determined to be between 4.5 and 6.0 and is identical to that of the enzyme secreted by C. albicans.

b) The temperature optimum of the S. cerevisiae secreted enzyme was found to be 60°C and is identical to that of the C. albicans secreted enzyme.

c) Aliquots of the culture medium derived from S. cerevisiae strain JCC4-11,

described above, transfomed with either YEp13 on pSTA27-1 and of C. albicans strain 792-1 were analyzed by SDS, polyacrylamide gel electrophoresis. A diffuse protein band of a molecular weight higher than 94K dalton is present in JCC4-11 (pSTA27-1) medium but absent from medium derived from JCC4-11 (YEp13). A similar, if not identical, protein band is detected, although in much lower amounts, in medium derived from C. albicans strain 792-1.

Taken together, these results indicate the fragment of C. albicans DNA cloned in pSTA27-1 carnies a C. albicans gene encoding a glucoamylase, that this gene is expressed in the yeast S. cerevisiae, and that the product of this gene is secreted into the culture medium by S. cerevisiae cells.

**Example 6**

Expression of the C. albicans galactokinase gene in S. cerevisiae

a. Isolation of the C. albicans galactokinase gene
The isolation of the C. albicans galactokinase gene was carried out according to the procedure of Example 1, except that S. cerevisiae strain JWG2-11D was employed as the host. Leu[+] transformants were plated on minimal media (Difco yeast nitrogen base) containing all required supplements except leucine and glucose, and also containing 2 % galactose.

b. Recovery of a plasmid containing the C. albicans galactokinase gene
Plasmids containing the C. albicans gene that codes for galactokinase were recovered in E: coli according to the procedure of Example 1. Plasmid DNA was isolated from individual E. coli transformants according to the method of Example 1.

c. Transformation of S. cerevisiae strain JWG2-11D with a plasmid containing the C. albicans galactokinase gene
The plasmid DNA was isolated from the E. coli transformants as described above and used to transform S. cerevisiae strain JWG2-11D according to the method of Example 1.

d. Selection of transformants and confirmation of the presence of C. albicans DNA in the transformant
Host S. cerevisiae microorganisms, transformed with a plasmid containing the C. albicans galactokinase gene acccording the method described above, were selected as described above, and the presence of the plasmid in the transformed host was confirmed according to the method of Example 1. Two independent isolates, designated as pCAgall-1 and pCAgall-4 were further analyzed by restriction mapping which revealed that the 7 kbp C. albicans DNA insert in pCAgall-1 is identical to the internal portion of the 13 kbp insert in pCAgall-4.

The presence of a C. albicans DNA sequence encoding galactokinase in the S. cerevisiae transformants was further demonstrated by measuring the level of galactokinase in strain JWG2-11D with on without plasmid pCAgall-4. Enzyme activity, measured by the method of Butt et al., PNAS-USA, 81, 3332 - 3336 (1984), was only detected in galactose induced cultures containing pCAgall-4.

The promoter of the C. albicans galactokinase gene was demonstrated to be positively activated by the S. cerevisiae positive regulator, the GAL-4 gene product, as follows. Strain JWG2-11D containing pCAgall-4 was mated to strain JWG2-22B (α-trpl-289, leu2-3, leu2-112, gall-152, ura3-52) containing plasmid pCD21-4 (a 2 μm Leu2 shuttle vector containing the S. cerevisiae GAL-4 gene). The resulting diploid was maintained on medium lacking both leucine and tryptophane. The induced level of galactokinase was significantly higher in the presence of the plasmid expressing the GAL-4 gene. This result indicates that the C. albicans sequence in pCAgall-4 contains a galactose regulated promoter which is responsive to S. cerevisiae regulatory proteins.

**Example 7**

Expression of the C. albicans isomaltase gene in S. cerevisiae

a. Isolation of the C. albicans isomaltase gene Isomaltase cleaves α-1-6 glucosidic bonds, and enables microorganisms expressing it to grow on the carbon source known as α-methyl-D-glucopyranoside.

The isolation of the C. albicans isomaltase gene was carried out according to the procedure of Example 1. Leu+ transformants were plated on minimal media (Difco - yease nitrogen base) containing all required supplements except leucine, and also containing 2 % α-methyl-D-glucopyranoside.

b. Recovery of a plasmid containing the C. albicans isomaltase gene

Plasmids containing the C. albicans gene that codes for isomaltase were recovered according to the procedure on Example 1. Plasmid DNA was isolated from individual E. coli transformants according to the method of example 1.

c. Transformation of S. cerevisiae strain JWG2-11D with a plasmid containing the C. albicans isomaltase gene

The plasmid DNA was isolated from the E. coli transformants described above and used to transform S. cerevisiae strain JWG2-11D according to the method of Example 1.

d. Selection of transformants and confirmation of the presence of C. albicans DNA in the transformant

Host S. cerevisiae microorganisms, transformed with a plasmid containing the C. albicans isomaltase gene according to the method described above, were selected as described above, and the presence of the plasmid in the transformed host was confirmed according to the method of Example 1.

**Example 8**

Transformation of S. cerevisiae with a recombinant plasmid containing a C. albicans replicator region

This example reports the isolation of several C. albicans replicator regions that support autonomous replication of recombinant plasmids in heterologous host S. cerevisiae microorganisms.

A. Materials and Methods

1. Strains and Media

Escherichia coli strain SF8 (C600, leuB, thr, pro, BI, rk⁻, mk⁻, recBC⁻, lopII, lig⁺) (Cameron et al PNAS, USA, 72, 3416 - 3420 (1975)], was used for bacterial transformation and propagation of plasmids. Bacteria were grown on LB (rich media) or M9 (minimal media) supplemented as required ((Millen, "Experiments In Molecular Genetics", Cold Spring Harbor Laboratory, New York, 431 - 433 (1972)]. Saccharomyces cerevisiae strain 483 (a, leu2-3, leu2-112, his4) was used as host for yeast transformation. Yeast cultures were grown on YPG (rich media 1 % yeast extract 1 % peptone, 2 % glucose) or YNB (minimal medium; Difco yeast nitrogen base) supplemented with histidine (2 μg/ml) and leucine (2 μg/ml) as required.

Plasmid M851 [Gorman et al., Mol. Gen. Genet., 183, 306-313 (1981)] was employed. C. albicans strain 792-1 was used as the source of genomic DNA.

2. DNA Preparations

Covalently closed circular plasmid DNA was prepared from M9-glucose growh E. coli cultures amplified with chloramphenicol. DNA was extracted and purified by a cleared lysate technique essentially as described by Clewell, J. Bacteriol., 110, 667 - 676 (1972).

Crude E. coli plasmid preparations, used for yeast transformation and preliminary gel analysis, were made using the procedure of Birnbohm and Doly, Nucl. Acids Res., 7, 1513 - 1523 (1979). Ten ml of bacterial culture (2x10¹⁰ cells) yielded 1-2 μg of crude plasmid DNA. For rapid screening of bacterial transformants for the presence of plasmid, single colony mini-lysates, prepared by the same method, were used.

For recovery of total DNA from S. cerevisiae, the small scale method of Sherman et al., cited above was employed.

C. albicans DNA was prepared by the method of Rigby et al., J. Mol. Biol., 111, 237 - 251 (1977).

3. Plasmid construction

Total Candida DNA was digested to completion with restriction endonuclease PstI. The DNA was mixed with PstI-cut M851 plasmid DNA at concentrations estimated to give maximal recovery of plasmids containing Candida inserts by the method of Dugaiczyk et al., J. Mol. Biol., 96, 171 - 184 (1975), and ligated with T4 polynucleotide ligase. The ligated mixture was used to transform E. coli strain SF8 to leucine prototrophy. Leu⁺ transformants were then screened for ampicillin sensitivity. The Ampˢ colonies (5.8 percent of the Leu + transformants), presumed to contain Candida inserts, were retained. The hybrid plasmids were designated by the prefix pCA followed by isolate number.

4. Gel Analysis and Hybridization procedures

All gel analysis and hybridization procedures employed were according to the method of Maniatis et al., cited above.

5. Transformation

E. coli were transformed using the calcium- heat shock method, as modified by Dagert and Ehrlich, Gene, 6, 23 - 28 (1979). The procedure used for yeast transformation was essentially as described by Beggs, Nature, 275, 104 - 109 (1978), using β-glucuronidase-sulfatase (Sigma) for spheroplast formation, with the exception that a lower concentration of plasmid DNA (0.5-10 μg per $10^9$ spheroplasts) was used.

B. Results

1. Plasmid construction

Candida DNA, digested to completion with restriction endonuclease PstI, was inserted by ligation into the unique PstI site of M851. Interruption of the $amp^r$ gene by an inserted fragment results in loss of drug resistance; thus $amp^S$ $Leu^+$ bacterial transformants were selected as harboring plasmids containing sequences from Candida (designated pCA).

2. Transformation of Yeast

Plasmids containing a yeast replicator sequence transform yeast at high frequency, reflecting autonomous replication of the plasmid in the eucaryotic cell. If the plasmid lacks a functional replicator, it also will transform, but at a low frequency. Presumably, this low-frequency stable transformation reflects integration of the plasmid $leu2^+$ gene into chromosomal DNA by homologous recombination. If Candida chromosomal replicators are functional in yeast, hybrid plasmids containing them might be characterized by the ability to transform yeast at high frequency.

Forty-one (41) pCA plasmids were individually tested for their ability to transform $leu2^-$ S. cerevisiae to $Leu^+$. Five of the forty-one pCA plasmids tested showed high frequency transformation. These results indicate that such plasmids can replicate autonomously in S. cerevisiae.

3. Detection of Plasmid DNA in S. cerevisiae

If high frequency transformation indicates the presence of a Candida sequence that allows autonomous plasmid replication, it should be possible to recover covalently closed circular molecules from the unstable $Leu^+$ yeast transformed with DNA from the pCA plasmids.

Total yeast DNA was extracted from parental and transformed yeast strains, electrophoresed in a 1.0 % agarose gel, and transferred to nitrocellulose paper.

The DNA was then hybridized with $P^{32}$-labelled pBR322 probe. This probe has no homology with yeast chromosomal DNA. The DNA from transformed cultures was transferred to nitrocellulose filters and hybridized with $p^{32}$-labelled pBR322- DNA sequences hybridizing to this probe could be detected in extracts of S. cerevisiae transformed with one of the five pCA plasmids giving high frequency transformation, as well as with control plasmid pYP42 (Gorman et al., cited above).

4. Transformation of E. coli with Yeast Plasmid DNA

The yeast plasmid preparations analyzed by hybridization techniques were also examined for ability to transform $leuB^-$ E. coli to $Leu^+$. Bacterial colonies selected on leucine-deficient medium were screened for the presence of plasmid by agarose gel electrophoresis of mini-lysates. The five yeast extracts that were shown to contain plasmids by hybridization gave rise to $Leu^+$ E. Coli that contained plasmid DNA. Plasmid DNA of one of the five high frequency transformation pCA plasmids was compared with the plasmid isolated from E. coli following transfer through the S. cerevisiae host. The plasmids, containing a 4.0 kilobase pain (kbp) C. albicans insert, were found to be identical.

Other replicaton regions of Candida useful in the autonomous malntainence of plasmids used to transform Saccharomyces can be isolated according to the method of Kawamuna et al., Gene, 24, 157 - 162 (1983), Hsu et al., J. Bacteriol., 154, 1033 - 1039 (1983), on Tikhomirova et al., Mol. Gen. Genet., 189, 479 - 485 (1983). Plasmids containing such replicator regions can be prepared according to the method described in this Example.

**Example 9**

Transformation of S. cerevisiae with a plasmid containing a C. albicans replicator region and a C. albicans coding sequence

A plasmid containing a C. albicans replicator region and a C. albicans coding sequence can be prepared by combining the methods of Examples 1 and 8. Such a plasmid can be used to transform S. cerevisiae according to the method of Example 1, and expression of the C. albicans coding sequence by the transformed S. cerevisiae host can be detected according to the method of Example 1.

While the above descriptions and Examples fully describe the invention and the preferred embodiments thereof, it is understood that the invention is not limited to the particular disclosed embodiments

Furthermore, this invention is not limited to the genes and promoters of Candida given in the examples above. Rather, this invention provides a general

21          EP 0 173 668 B2          22

method for locating Candida gene expression units, promoters, regulatory regions and structural genes, as well as a genenal method for expressing such functional DNA sequences in Saccharomyces.

Thus, the invention includes all embodiments coming within the scope of the following claims.

## Claims

**Claims for the following Contracting States : BE, CH, DE, FR, GB, IT, LI, LU, NL, SE**

1. A process for preparing a transformed host microorganism of the genus Saccharomyces which comprises transforming said host microorganism with a functional DNA sequence derived from Candida albicans, wherein the functional DNA sequence comprises a complete gene expression unit, a structural gene, a promoter, or a regulatory region.

2. The process of Claim 1 wherein the functional DNA sequence is derived from a trp1 gene, a his3, gene, a gene conferring resistance to the anti-fungal agent benomyl, a functional DNA sequence allowing for growth on the carbon source sorbitol, a functional DNA sequence allowing for growth on soluble starch, a functional DNA sequence allowing for growth on isomaltose, or a galactokinase gene

3. The process of Claim 1 wherein the Saccharomyces belongs to the species Saccharomyces cerevisiae.

4. The process of Claim 1 which comprises effecting the transformation with a recombinant plasmid, wherein said plasmid comprises a replicator region and the functional DNA sequence derived from Candida albicans.

5. The process of Claim 4 wherein the replicator region-is derived from Saccharomyces or Candida albicans.

6. The process of Claim 4 wherein the recombinant plasmid is a Saccharomyces-Escherichia Candida albicans shuttle vector.

7. The process of Claim 6 wherein the shuttle vector is a YEp13 derivative.

8. A process for preparing a recombinant plasmid comprising a replicator region, and a functional DNA sequence derived from Candida albicans, which comprises ligating said replicator region directly or indirectly with the functional DNA sequence, wherein the functional DNA sequence comprises a complete gene expression unit, a structural gene, a promoter, or a regulatory region.

9. The process of claim 8 wherein the replicator region is derived from Saccharomyces or Candida albicans.

10. The process of Claim 8 wherein the functional DNA sequence is derived from a trp1 gene, a his3 gene, a gene conferring resistance to the anti-fungal agent benomyl, a gene allowing for growth on the carbon source sorbitol, a glucoamylase gene, an isomaltase gene, or a galactokinase gene.

11. The process of claim 8 wherein the recombinant plasmid is a Saccharomyces-Escherichia coli shuttle vector.

12. The process of Claim 11 wherein the shuttle vector is a YEp13 derivative.

13. A method of expressing a functional DNA sequence derived from Candida albicans which comprises transforming a host microorganism of the genus saccharomyces with the functional DNA sequence, and culturing the transformed host under suitable conditions such that the functional DNA sequence is expressed, wherein the functional DNA sequence comprises a complete gene expression unit, a structural gene, a promoter, or a regulatory region.

14. The method according to Claim 13 wherein the functional DNA sequence is derived from a trp1 gene, a his3 gene conferring resistance to the antifungal agent benomyl, a gene allowing for growth on the carbon source sorbitol, a glucoamylase gene, an isomaltase gene, or a galactokinase gene.

15. The method according to Claim 13 wherein the host microorganism belongs to the species Saccharomyces cerevisiae.

16. The method according to Claim 13 which further comprises effecting the transformation with a recombinant plasmid, wherein said plasmid comprises a replicator region, and the functional DNA sequence derived from Candida albicans.

17. The method according to Claim 16 wherein the replicator region is derived from Saccharomyces or Candida albicans.

18. The method according to Claim 16 wherein the recombinant plasmid is a Saccharomyces-Escherichia coli shuttle vector.

19. The method according to Claim 18 wherein the shuttle vector is a YEp13 derivative.

20. A method of characterising a DNA sequence derived from Candida albican by its function which comprises (a) transforming a host microorganism of the genus Saccharomyces with the DNA sequence, wherein said host microorganism lacks a known function, so that the Candida albicans DNA can complement the host microorganism's DNA; and (b) assaying for the presence of the function in the transformed host microorganism.

21. A method of producing a polypeptide in Saccharomyces which comprises culturing a transformed host microorganism of the genus Saccharomyces, wherein said host microorganism comprises a promoter derived from Candida albicans fused, directly or indirectly, in phase and in proper orientation to a structural gene which codes for the polypeptide.

12

Claims for the following Contracting State:
AT

1. A method of expressing a functional DNA sequence derived from Candida albicans which comprises transforming a host microorganism of the genus Saccharomyces with functional DNA sequence, and culturing the transformed host under suitable conditions such that the functional DNA sequence is expressed, wherein the functional DNA sequence comprises a complete gene expression unit, a structural gene, a promoter, or a regulatory region.

2. The method according to Claim **1** wherein the functional DNA sequence is derived from a trp1 gene, a his3 gene, a gene conferring resistance to the antifungal agent benomyl, a gene allowing for growth on the carbon source sorbitol, a glucoamylase gene, an isomaltase gene, or a galactokinase gene.

3. The method according to Claim 1 wherein the host microorganism belongs to the species Saccharomyces cevevisiae.

4. The method according to Claim 1 which further comprises effecting the transformation with a recombinant plasmid, wherein said plasmid comprises a replicator region, and the functional DNA sequence derived from Candida albicans.

5. The method according to Claim 4 wherein the replicator region is derived from Saccharomyces or Candida albicans.

6. The method according to Claim 4 wherein the recombinant plasmid is a Saccharomyces -Escherichia coli shuttle vector.

7. The method according to Claim 6 wherein the shuttle vector is a YEp13 derivative.

8. A method of characterising a DNA sequence derived from Candida albicans by its function which comprises (a) transforming a host microorganism of the genus Saccharomyces with the DNA sequence, wherein said host microorganism lacks a known function, so that the Candida albicans DNA can complement the host microorganism's DNA; and (b) assaying for the presence of the function in the transformed host microorganism.

9. A method of producing a polypeptide in Saccharomyces which comprises culturing a transformed host microorganism of the genus Saccharomyces, wherein said host microorganism comprises a promoter derived from Candida albicans fused, directly or indirectly, in phase and in proper orientation to a structural gene which codes for the polypetide.

Patentansprüche

Patentansprüche für folgenden Vertragsstaaten: BE, CH, DE, FR, GB, IT, LI, LU, NL, SE

1. Verfahren zur Herstellung eines transformierten Wirts-Mikroorganismus der Gattung Saccharmyces, umfassend die Transformation des Wirts-Mikroorganismus mit einer von Candida albicans stammenden funktionellen DNA-Sequenz, wobei die funktionelle DNA-Sequenz eine vollständige Gen-Expressionseinheit, ein Strukturgen, einen Promotor und einen Steuerbereich umfaßt.

2. Verfahren nach Anspruch 1, wobei die funktionelle DNA-Sequenz von einem trp1-Gen, einem his3-Gen, einem Resistenz gegen den Antipilz-wirkstoff Benomyl erzeugenden Gen, einer das wachstum auf der Kohlenstoffquelle Sorbit ermöglichenden funktionellen DNA-Sequenz, einer das Wachstum auf löslicher Stärke ermöglichenden funktionellen DNA-Sequenz, einer das Wachstum auf Isomaltose ermöglichenden funktionellen DNA-Sequenz oder einem Galac tokinase-Gen stammt.

3. Verfahren nach Anspruch 1, wobei der Saccharomyces zur Art Saccharomyces cerevisiae gehört.

4. Verfahren nach Anspruch 1, welches die Durchführung der Transformation mit einem rekombinanten Plasmid umfaßt, wobei das Plasmid einen Replikationsbereich und die von Candida albicans stammende funktionelle DNA-Sequenz um faßt.

5. Verfahren nach Anspruch 4, wobei der Replikationsbereich von Saccharomyces oder Candida albicans stammt.

6. Verfahren nach Anspruch 4, wobei das rekombinante Plasmid ein saccharomyces-Escherichia coli-Shuttle-vektor ist.

7. verfahren nach Anspruch 6, wobei der Shuttle-vektor ein YEp13-Derivat ist.

8. verfahren zur Herstellung eines rekombinanten Plasmids, das einen Replikationsbereich und eine von Candida albicans stammende funktionelle DNA-Sequenz umfaßt, das die direkte oder indirekte Ligierung des Replikationsbereiches mit der funktionellen DNA-Sequenz umfaßt, wobei die funktionelle DNA-Sequenz eine vollständige Gen-Ex pressïonseinheit, ein Strukturgen, einen Promotor oder einen Steuerbereich umfaßt.

9. verfahren noch Anspruch 8, wobei der Replikationsbereich von Saccharomyces oder Candida albicans stammt.

10. verfahren nach Anspruch 8, wobei die funktionelle DNA-Sequenz von einem trp1-Gen, einen his3-Gen, einem Resistenz gegen den Antipilz-Wirkstoff Benomyl erzeugenden Gen, einem das Wachstum auf der Kohlenstoffquelle Sorbit ermöglichenden Gen, einem Glucoamylase-Gen, einem Isomaltase-Gen oder einem Galactokinase-Gen stammt.

11. verfahren nach Anspruch 8, wobei das rekombinante Plasmid ein Saccharomyces-Escherichia coli-Shuttle-vektor ist.

12. verfahren nach Anspruch 11, wobei der Shuttle-vektor ein YEp13-Derivat ist.

13. verfahren zur Expression einer von Candida albicans stammenden funktionellen DNA-Sequenz, das die Transformation eines Wirts-Mikroorganismus der Gattung Saccharomyces mit der funktionellen DNA-Sequenz und die Züchtung des transformierten Wirtes unter geeigneten Bedingungen umfaßt, so daß die funktionelle DNA-Sequenz exprimiert wird, wobei die funktionelle DNA-Sequenz eine vollständige Gen-Expressionseinheit, ein Strukturgen, einen Promotor oder einen Steuerbereich umfaßt.

14. Verfahren nach Anspruch 13, wobei die funktionelle DNA-Sequenz von einem trp1-Gen, einem his3-Gen, einem Resistenz gegen den Antipilz-wirkstoff Benomyl erzeugenden Gen, einem das Wachstum auf der Kohlenstoffquelle Sorbit ermöglichenden Gen, einem Glucoamylase-Gen, einem Isomaltase-Gen oder einem Galactokinase-Gen stammt.

15. Verfahren nach Anspruch 13, wobei der Wirts-Mikroorganismus zur Art Saccharomyces cerevisiae gehört.

16. Verfahren nach Anspruch 13, welches ferner die Durchführung der Transformation mit einem rekombinanten Plasmid umfaßt, wobei das Plasmid einen Replikationsbereich und die von Candida albicans stammende funktionelle DNA-Sequenz umfaßt.

17. Verfahren nach Anspruch 16, wobei der Replikationsbereich von Saccharomyces oder Candida albicans stammt.

18. Verfahren nach Anspruch 16, wobei das rekombinante Plasmid ein Saccharomyces-Escherichia coli-Shuttle-Vektor ist.

19. Verfahren nach Anspruch 18, wobei der Shuttle-Vektor ein YEp13-Derivat ist.

20. Verfahren zur Charakterisierung einer von Candida albicans stammenden DNA-Sequenz durch ihre Funktion, umfassend (a) die Transformation eines Wirts-Mikroorganismus der Gattung Saccharomyces mit der DNA-Sequenz, wobei der Wirts-Mikroorganismus eine bekannte Funktion nicht besitzt, so daß die Candida albicans-DNA die DNA des Wirts-Mikroorganismus vervollständigen kann, und (b) die Durchführung eines Tests auf die Anwesenheit der Funktion in dem transformierten Wirts-Mikroorganismus.

21. Verfahren zur Erzeugung eines Polypeptids in Saccharomyces, das die Züchtung einem transformierten Wirts-Mikroorganismus der Gattung Saccharomyces umfaßt, wobei der Wirts-Mikroorganismus einen von Candida albicans stammenden Promotor umfaßt, der direkt oder indirekt in Phase und in richtiger Orientierung an ein Strukturgen fusioniert ist, das das Polypeptid codiert.

**Patentansprüche für folgenden Vertragsstaat: AT**

1. Verfahren zur Expression einer von Candida albicans stammenden funktionellen DNA-Sequenz, das die Transformation eines Wirts-Mikroorganismus der Gattung Saccharomyces mit der funktionellen DNA-Sequenz und Züchtung des transformierten Wirts unter geeigneten Bedingungen umfaßt, so daß die funktionelle DNA-sequenz exprimiert wird, wobei die funktionelle DNA-sequenz eine vollständige Gen-Expressionseinheit, ein Strukturgen, einen Promotor oder einen steuerbereich umfaßt.

2. Verfahren nach Anspruch 1, wobei die funktionelle DNA-Sequenz von einem trp1-Gen, einem his3-Gen, einem Resistenz gegen den Antipilz-Wirkstoff Benomyl erzeugenden Gen, einem das Wachstum auf der Kohlenstoffquelle Sorbit ermöglichenden Gen, einem Glucoamylase-Gen, einem Isomaltase-Gen oder einem Galactokinase-Gen stammt.

3. Verfahren nach Anspruch 1, wobei der Wirts-Mikroorganismus zur Art Saccharomyces cevevisiae gehört.

4. Verfahren nach Anspruch 1, welches ferner die Durchführung der Transformation mit einem rekombinanten Plasmid umfaßt, wobei das Plasmid einen Replikationsbereich und die von Candida albicans stammende funktionelle DNA-Sequenz umfaßt.

5. Verfahren nach Anspruch 4, wobei der Replikationsbereich von Saccharomyces oder Candida albicans stammt.

6. Verfahren nach Anspruch 4, wobei das rekombinante Plasmid ein Saccharomyces-Escherichia coli-Shuttle-Vektor ist.

7. Verfahren nach Anspruch 6, wobei der Shuttle-Vektor ein YEp13-Derivat ist.

8. Verfahren zur Charakterisierung einer von Candida albicans stammenden DNA-Sequenz durch ihre Punktion, umfansend (a) die Transformation eines Wirts-Mikroorganismus der Gottung Saccharomyces, mit der DNA-Sequenz, wobei der Wirts-Mikroorganismus eine bekannte Punktion nicht besitzt, so daß die Candida albicans-DNA die DNA des Wirts-Mikroorganismus vervollständigen kann, und (b) die Durchführung eines Tests auf die Anwesenheit der Funktion in dem transformierten Wirts-Mikroorganismus.

9. Verfahren zur Erzeugung eines Polypeptids in Saccharomyces, das die Züchtung eines transformierten Wirts-Mikroorganismus der Gattung Saccharomyces, umfaßt, wobei der Wirts-Mikroorganismus einen von Candida albicans stammenden Promotor umfaßt, der direkt oder indirekt in Phase und in richtiger Orientierung an ein Strukturgen fusioniert ist, das das Polypeptid codiert.

## Revendications

### Revendications pour les Etats contractants suivants: BE, CH, DE, FR, GB, IT, LI, LU, NL, SE

1. Procédé pour préparer un microorganisme hôte transformé du genre Saccharomyces qui comprend la transformation de ce microorganisme hôte avec une séquence d'ADN fonctionnelle dérivée de Candida albicans, dans lequel la séquence d'ADN fonctionnelle comprend une unité d'expression de gène complète, un gène structural, un promoteur ou une région régulatrice.

2. Procédé suivant la revendication 1, dans lequel la séquence d'ADN fonctionnelle provient d'un gène trp1, d'un gène his3, d'un gène conférant une résistance au bénomyle, un agent antifongique d'une séquence d'ADN fonctionnelle permettant une croissance sur le sorbitol comme source de carbone, d'une séquence d'ADN fonctionnelle permettant une croissance sur un amidon soluble, d'une séquence d'ADN fonctionnelle permettant la croissance sur de l'isomaltose, ou un gène de galactokinase.

3. Procédé suivant la revendication 1, dans lequel le Saccharomyces appartient à l'espèce Saccharomyces cerevisiae.

4. Procédé suivant la revendication 1, qui comprend la réalisation de la transformation avec un plasmide recombinant, dans lequel ce plasmide comprend une région de réplication et la séquence d'ADN fonctionnelle provient de Candida albicans.

5. Procédé suivant la revendication 4, dans lequel la région de réplication provient de Saccharomyces ou de Candida albicans.

6. Procédé suivant la revendication 4, dans lequel le plasmide recombinant est un vecteur navette Saccharomyces-Escherichia coli.

7. Procédé suivant la revendication 6, dans lequel le vecteur navette est un dérivé YEp13.

8. Procédé pour préparer un plasmide recombinant comprenant une région de réplication et une séquence d'ADN fonctionnelle provenant de Candida albicans, qui comprend la ligature de cette région de réplication directement ou indirectement avec la séquence d'ADN fonctionnelle, dans lequel la séquence d'ADN fonctionnelle comprend une unité d'expression de gène complète, un gène structural, un promoteur ou une région régulatrice.

9. Procédé suivant la revendication 8, dans lequel la région de réplication provient de Saccharomyces ou de Candida albicans.

10. Procédé suivant la revendication 8, dans lequel la séquence d'ADN fonctionnelle provient d'un gène trp1, d'un gène His3, d'un gène conférant une résistance au bénomyle, un agent antifongique, d'un gène permettant une croissance sur le sorbitol comme source de carbone, d'un gène de glucoamylase, d'un gène d'isomaltase ou d'un gène de galactokinase.

11. Procédé suivant la revendication 8, dans lequel le plasmide recombinant est un vecteur navette Saccharomyces-Escherichia coli.

12. Procédé suivant la revendication 11, dans lequel le vecteur navette est un dérivé direct de YEp13.

13. Méthode pour exprimer une séquence d'ADN fonctionnelle provenant de Candida albicans, qui comprend la transformation d'un microorganisme hôte du genre Saccharomyces avec la séquence d'ADN fonctionnelle et la culture de l'hôte transformé dans des conditions convenables de façon à exprimer la séquence d'ADN fonctionnelle, dans laquelle la séquence d'ADN fonctionnelle comprend une unité d'expression de gène complète, un gène structural, un promoteur ou une région régulatrice.

14. Méthode suivant la revendication 13, dans laquelle la séquence d'ADN fonctionnelle dérive d'un gène trp1, d'un gène his3, d'un gène conférant une résistance au bénomyle, un agent antifongique, d'un gène permettant une croissance sur le sorbitol comme source de carbone, d'un gène de glucoamylase, d'un gène d'isomaltase ou d'un gène de galactokinase.

15.- Méthode suivant la revendication 13, dans laquelle le microorganisme hôte appartient à l'espèce Saccharomyces cerevisiae.

16. Méthode suivant la revendication 13, qui comprend de plus la réalisation de la transformation avec un plasmide recombinant, dans laquelle ce plasmide comprend une région de réplication et la séquence d'ADN fonctionnelle provient de Candida albicans.

17. Méthode suivant la revendication 16, dans laquelle la région de réplication provient de Saccharomyces ou de Candida albicans.

18. Méthode suivant la revendication 16, dans laquelle le plasmide recombinant est un vecteur navette Saccharomyces-Escherichia coli.

19. Méthode suivant la revendication 18, dans laquelle le vecteur navette est un dérivé YEp13.

20. Méthode pour caractériser une séquence d'ADN dérivée de Candida albicans par sa fonction, qui comprend

(a) la transformation d'un microorganisme hôte du genre Saccharomyces avec la séquence d'ADN, dans laquelle ce microorganisme hôte manque d'une fonction connue de telle sorte que l'ADN de Candida albicans peut compléter l'ADN du microorganisme hôte ; et

(b) laissé , pour déterminer la présence de la fonction dans le microorganisme hôte transformé.

21. Méthode pour produire un polypeptide dans Saccharomyces qui comprend la culture d'un miroorganisme hôte transformé du genre Saccharomyces, dans laquelle ce microorganisme hôte comprend un promoteur provenant de Candida albicans fusionné directement ou indirectement en phase et en orientation convenables à un gène strutural qui code pour le polypeptide.

**Revendications pour l'Etat contractant suivant: AT**

1. Méthode pour exprimer une séquence d'ADN fonctionnelle provenant de Candida albicans qui comprend la transformation d'un microorganisme hôte du genre Saccharomyces avec une séquence d'ADN fonctionnelle et la culture de l'hôte transformé dans des conditions convenables de façon à exprimer la séquence d'ADN fonctionnelle, dans laquelle la séquence d'ADN fonctionnelle comprend une unité d'expression de gène complète, un gène structural, un promoteur ou une région régulatrice.

2. Méthode suivant la revendication 1, dans laquelle la séquence d'ADN fonctionnelle provient d'un gène trp1, d'un gène His3, d'un gène conférant une résistance au bénomyle, un agent antifongique, d'un gène permettant une croissance sur le sorbitol comme source de carbone, d'un gène de glucoamylase, d'un gène d'isomaltase ou d'un gène de galactokinase.

3. Méthode suivant la revendication 1, dans laquelle le microorganisme hôte appartient à l'espèce Saccharomyces cerevisiae.

4. Méthode suivant la revendication 1, qui comprend de plus la réalisation de la transformation avec un plasmide recombinant, dans laquelle ce plasmide comprend une région de réplication et la séquence d'ADN fonctionnelle provient de Candida albicans.

5. Méthode suivant la revendication 4, dans laquelle la région de réplication provient de Saccharomyces ou de Candida albicans.

6. Méthode suivant la revendication 4, dans laquelle le plasmide recombinant est un vecteur navette Saccharomyces-Escherichia coli.

7. Méthode suivant la revendication 6, dans laquelle le vecteur navette est un dérivé YEp13.

8. Méthode pour caractériser une séquence d'ADN provenant de Candida albicans par sa fonction qui comprend :

(a) la transformation d'un microorganisme hôte du genre Saccharomyces avec la séquence d'ADN, dans laquelle ce microorganisme hôte manque d'une fonction connue de telle sorte que l'ADN de Candida albicans peut compléter l'ADN du microorganisme hôte ; et

(b) laissé pour déterminer la présence de la fonction dans le microorganisme hôte transformé.

9. Méthode pour produire un polypeptide dans Saccharomyces qui comprend la culture d'un microorganisme hôte transformé du genre Saccharomyces, dans laquelle ce microorganisme hôte comprend un promoteur provenant de Candida albicans fusionné directement ou indirectement, en phase et dans l'orientation convenable à un gène structural qui code pour le polypeptide.